# EUROPEAN PATENT SPECIFICATION

(11) **EP 2 906 536 B1**
(45) Date of publication and mention of the grant of the patent: **14.03.2018**
(21) Application number: 13845500.1
(22) Date of filing: 04.10.2013
(51) Int. Cl.: C07D 209/42, C07K 5/08

(54) **AN IMPROVED PROCESS FOR PREPARATION OF PERINDOPRIL INTERMEDIATE**
VERBESSERTES VERFAHREN ZUR HERSTELLUNG EINES PERINDOPRIL-ZWISCHENPRODUKTS
PROCÉDÉ AMÉLIORÉ DE PRÉPARATION D'UN INTERMÉDIAIRE DE PÉRINDOPRIL

(30) Priority: 10.10.2012 IN 2961MU2012
(43) Date of publication of application: 19.08.2015
(73) Proprietor: Piramal Enterprises Limited, Mumbai 400 013 (IN)
(72) Inventor: WAGH, Ganesh, Mumbai 400063 (IN); JAGTAP, Ashutosh, Mumbai 400063 (IN); ROY, Mita, Mumbai 400012 (IN); HARIHARAN, Sivaramakrishnan, Egattur Chennai 603103 (IN)
(74) Representative: Richards, Michèle E.
(86) International application number: PCT/IB2013/059112
(87) International publication number: WO 2014/057404

(56) References cited:
- EP-B9- 1 679 072
- WO-A1-2006/131828
- WO-A1-2008/114270
- US-A1- 2006 178 422

## Description

### Field of the invention

The present invention relates to a process for the preparation of a key intermediate used in the synthesis of (2S,3aS,7aS)-1-[(2S)-2-[[(1S)-1-(ethoxycarbonyl) butyl]amino]-1-oxopropyl]octahydro-1H-indole-2-carboxylic acid (Perindopril or the compound of formula I) and its pharmaceutically acceptable salt, particularly tert-butylamine salt (Perindopril erbumine). More particularly, the present invention relates to a process for the preparation of (2S,3aS,7aS)-1-[(2S)-2-[[(1S)-1-(ethoxycarbonyl)butyl]amino]-1-oxopropyl]octahydro-1H-indole-2-carboxylic acid benzyl ester (the compound of formula II), the key intermediate for the synthesis of the compound of formula I (Perindopril) and its tert-butylamine salt (Perindopril erbumine).

### Background of the invention

Perindopril, (2S,3aS,7aS)-1-[(2S)-2-[[(1S)-1-(ethoxycarbonyl)butyl]amino]-1-oxopropyl]octahydro-1H-indole-2-carboxylic acid is represented by following formula I (hereinafter referred as "the compound of formula I").

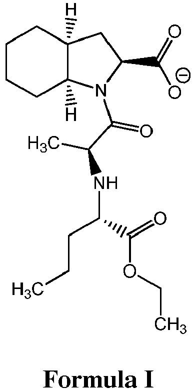

Perindopril and its tert-butylamine salt (Perindopril erbumine) are long acting angiotensin-converting-enzyme inhibitor (ACE inhibitor). Perindopril is used to treat hypertension, heart failure or stable coronary artery disease. Perindopril is the free acid form of Perindopril erbumine. Perindopril is a pro-drug which is metabolized *in vivo* by hydrolysis of the ester group to form perindoprilat, a biologically active metabolite. The mechanism through which perindoprilat lowers the blood pressure is believed to be primarily due to inhibition of ACE activity. ACE is a peptidyl dipeptidase that catalyzes conversion of the inactive decapaptide, angiotensin I, to the vasoconstrictor, angiotensin II. Inhibition of ACE results in decreased plasma angiotensin II, leading to decreased vasoconstriction, increased plasma renin activity and decreased aldosterone secretion. Perindopril erbumine is marketed under brand name Aceon®.

(2S,3aS,7aS)-1-[(2S)-2-[[(1S)-1-(ethoxycarbonyl)butyl]amino]-1-oxopropyl]octahydro-1H-indole-2-carboxylic acid benzyl ester (hereinafter referred as "the compound of formula II") is a key intermediate used for the synthesis of the compound of formula I (Perindopril) and its tert-butylamine salt.

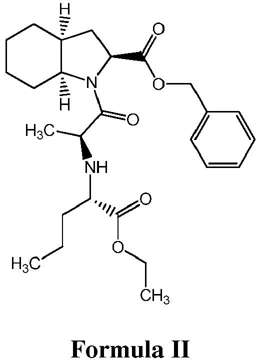

A process for the preparation of the compound of formula II is described in US Patent No. 4914214. This process involves the reaction of (2S,3aS,7aS)-octahydro-1H-indole-2-carboxylic acid phenylmethyl ester 4-methylbenzenesulfonate (hereinafter referred as "the compound of formula III") with N-[(S)-ethoxycarbonyl-1-butyl]-(S)-alanine (hereinafter referred as "the compound of formula IV") using triethylamine, dicyclohexylcarbodiimide (DCC) and 1-hydroxybenzotriazole (HOBT) in the presence ethyl acetate as a solvent for 3 hours. After completion of the reaction, dicyclohexylurea (DCU) is precipitated out as a by-product, which is then filtered off. The filtrate is then collected and washed with water and further evaporated to dryness to yield the compound of formula II. The compound of formula II is further debenzylated to give the compound of formula I which is further converted to its tert-butylamine salt. The process disclosed in said patent involves use of ethyl acetate as the solvent. The use of ethyl acetate as the solvent generates one major impurity viz (2S,3aS,7aS)-1-acetyloctahydro-1H-indole-2-carboxylic acid benzyl ester. Further use of dicyclohexylcarbodiimide (DCC) leads to formation of dicyclohexylurea (DCU) as a by-product. However, due to use of ethyl acetate as a solvent the by-product as formed does not get removed completely.

US patent no. 7279583 describes a process for the preparation of the compound of formula II comprising the reaction of the compound of formula III with the compound of formula IV using triethylamine and O-(benzotriazol-1-yl)-1,1,3,3-bis-(tetramethylene)uronium hexafluorophosphate in the presence of ethyl acetate as a solvent. The resulting heterogeneous mixture is heated at a temperature of 30°C for 3 hours and then cooled at a temperature of 0°C. The reaction mixture is then filtered and the filtrate as obtained is evaporated to dryness to yield the compound of formula II.

PCT patent application no. WO2008/114270 describes a process for the preparation of the compound of formula II comprising the reaction of the compound of formula III with the compound of formula IV using triethylamine, 1-hydroxybenzotriazole (HOBT) and dicyclohexylcarbodimide (DCC) in the presence of isopropyl acetate as a solvent at a temperature of 25°C. The resulting reaction mixture is then cooled to a temperature of 5°C to 10°C to precipitate a by-product namely dicyclohexylurea (DCU). The precipitated by-product is then filtered off. The filtrate is then collected and washed with water and further evaporated to dryness to yield the compound of formula II.

Indian patent no. 231512 describes a process for the preparation of the compound of the formula II comprising the reaction of the compound of formula III with the compound of formula IV using triethylamine, 1-hydroxybenzotriazole (HOBT) and dicyclohexylcarbodiimide (DCC) in the presence of inert solvent other than ethyl acetate such as methylene chloride, chloroform, acetonitrile, N,N-dimethylformamide and cyclohexane to obtain the heterogeneous mass, which is then stirred at a temperature of 20°C to 25°C. After completion of the reaction the by-product dicyclohexylurea (DCU) is precipitated out. The precipitated by-product is filtered and the filtrate is distilled under vacuum to obtain the crude compound of formula II. This crude compound of formula II contains traces of dicyclohexylurea (DCU) which is removed by further purification in diisopropyl ether. The process disclosed in said patent involves purification of the compound of formula II. Thus, the process involves numerous steps and long reaction time which renders the process costlier and industrially not viable.

In summary, the processes for the preparation of the compound of formula II as disclosed in prior art primarily used ethyl acetate as a solvent which involved reaction of the compound of formula III with the compound of formula IV. Use of ethyl acetate as a reaction solvent generates an impurity namely (2S,3aS,7aS)-1-acetyloctahydro-1H-indole-2-carboxylic acid benzyl ester of formula V (hereinafter referred to as 'N-acetyl impurity').

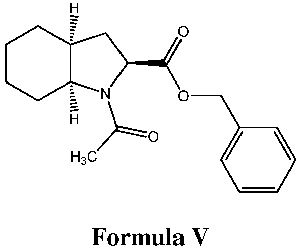

The presence of ethyl acetate in the reaction mixture acts as an acylating agent to form the N-acetyl impurity. The removal of N-acetyl impurity by purification method is very difficult as the said impurity and the compound of formula II are similar in respect of their properties. It is also difficult to remove the said impurity when the compound of formula II is debenzylated to give the compound of formula I because during debenzylation of the compound of formula II, said N-acetyl impurity also gets debenzylated to give the corresponding N-acetyl(2S,3aS,7aS)octahydroindole-2-carboxylic acid of formula VI, as an impurity.

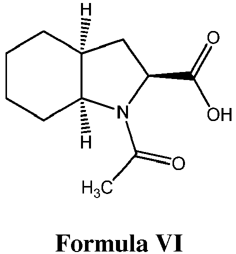

Further during the salt formation of the compound of formula I with tert-butylamine to form Perindopril erbumine, the compound of formula VI invariably remains contaminated with the product, Perindopril erbumine. Thus, it is difficult to remove N-acetyl impurity by purification method. Therefore, there is a need to develop an improved process for the preparation of the compound of formula II that would avoid formation of N-acetyl impurity.

Another major drawback of the prior art processes for the preparation of the compound of formula II involving reaction of the compound of formula III with the compound of formula IV is that the by-product dicyclohexyl urea (DCU) is not completely removed. The process for the preparation of the compound of formula II involving the reaction of the compound of formula III with the compound of formula IV uses dicyclohexylcarbodiimide (DCC) as a reagent which reacts with the compound of formula IV resulting in the formation of dicyclohexylurea (DCU) as a by-product. This by-product that is formed in the reaction mixture is required to be filtered off. The filtrate as obtained is then concentrated to give the compound of formula II. However, in the given reaction condition the traces of dicyclohexylurea (DCU) continue to remain in the filtrate. This filtrate is further concentrated to give the compound of formula II having traces of dicyclohexylurea (DCU). The process disclosed in prior art requires an additional purification of the compound of formula II using diisopropyl ether or cyclohexane or the mixture thereof for removal of dicyclohexylurea (DCU). Therefore, additional purification step is required to remove traces of dicyclohexylurea (DCU). Thus, there is a need to develop an improved process for the preparation of the compound of formula II to avoid additional step of purification for removal of the by-product, dicyclohexylurea (DCU) thereby making the process industrially viable.

The inventors of the present invention have now found that the compound of formula II free of the impurities namely N-acetyl impurity and dicyclohexylurea (DCU) can be obtained in good yield and enhanced purity through an improved process involving the reaction of the compound of formula III with the compound of formula IV in toluene as a reaction solvent.

### Objects of the invention

An object of the present invention is to provide an improved process for the preparation of the compound of formula II (as described herein) comprising reacting the compound of formula III with the compound of formula IV using toluene as a reaction solvent.

Another object of the present invention is to provide an improved process for the preparation of the compound of formula II, wherein said process avoids formation of N-acetyl impurity.

Yet another object of the present invention is to provide an improved process for the preparation of the compound of formula II, wherein said process involves complete removal of the by-product, dicyclohexylurea (DCU).

Further object of the present invention is to provide an improved process for the preparation of the compound of formula II with yield of 99% and purity of 95%, wherein said process does not involve an additional purification step.

### Summary of the invention

In accordance with an aspect of the present invention, there is provided an improved process for the preparation of the compound of formula II comprising reacting the compound of formula III with the compound of formula IV using 1-hydroxybenzotriazole (HOBT), dicyclohexylcarbodiimide (DCC) and triethylamine in the presence of toluene as a solvent at a temperature of 5°C to 40°C.

The process of the present invention is depicted in scheme I herein below:

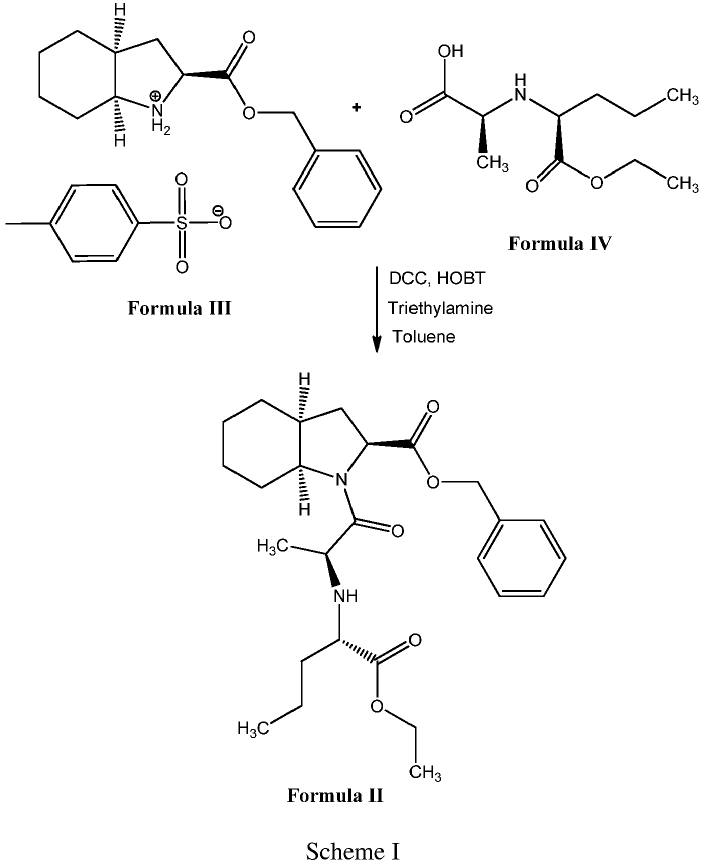

Importantly, the process for the preparation of the compound of formula II involving reaction of the compound of formula III with the compound of formula IV using toluene as a solvent avoids formation of N-acetyl impurity.

Also, the process for the preparation of the compound of formula II involving reaction of the compound of formula III with the compound of formula IV using toluene as a solvent avoids additional step of purification required for the complete removal of byproduct, dicylohexylurea (DCU).

The compound of formula II prepared according to the present process is obtained in a yield of 99% and a purity of 95%.

### Detail description of the invention

Accordingly, the present invention relates to an improved process for the preparation of the compound of formula II.

According to the present invention, the improved process for the preparation of the compound of formula II involves use of toluene as a solvent.

Thus, the present invention relates to a process for the preparation of the compound of formula II, 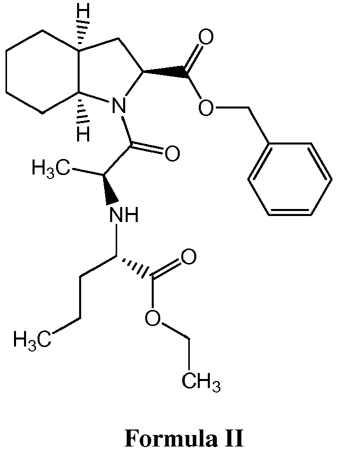 comprising,
reacting the compound of formula III, 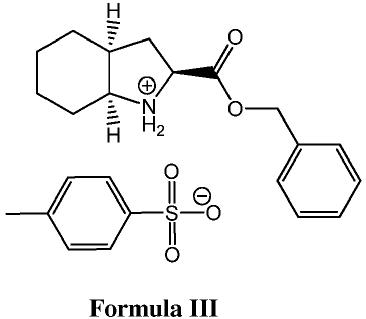 with the compound of formula IV, 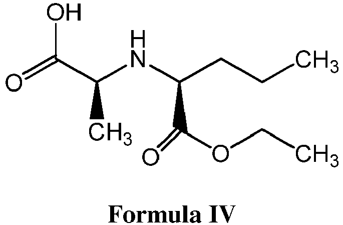 using 1-hydroxybenzotriazole (HOBT), dicyclohexylcarbodiimide (DCC) and triethylamine in the presence of toluene as a solvent at a temperature of 5- 40°C to obtain the compound of formula II.

In accordance with an embodiment of the present invention, the solvent, toluene is used in an amount ranging from 10 to 15 volumes based on the compound of formula III.

In accordance with an embodiment of the present invention, the reaction is carried out at a temperature of 15-20°C.

In accordance with an embodiment of the present invention, triethylamine is used in an amount ranging from 1 to 3 molar equivalents based on the compound of formula III.

In accordance with an embodiment of the present invention, 1-hydroxybenzotriazole (HOBT) is used in an amount ranging from 0.8 to 2.5 molar equivalents based on the compound of formula III.

In accordance with an embodiment of the present invention, dicyclohexylcarbodiimide (DCC) is used in an amount ranging from 0.8 to 2.5 molar equivalents based on the compound of formula III.

The starting material of the process, i.e. the compound of formula III is a known compound and can be prepared by a person skilled in the art by following the methods described in the literature. For example, the process described in the US Patent No. 4914214 can be used for the preparation of the compound of formula III. The process involves reacting (2S,3aS,7aS)-2-carboxyperhydroindole with 4-methylbenzenesulfonate and benzyl alcohol using toluene as a solvent. The water formed during the reaction is removed by azeotropic distillation. After complete removal of water, the reaction mixture is cooled to precipitate the compound of formula III.

According to the present invention, the process for the preparation of the compound of formula II involves charging of the compound of formula III, toluene and triethylamine to the reaction flask and the reaction mixture was stirred at a temperature of 20-30°C for 30 to 60 minutes. To the resulting reaction mixture the compound of formula IV, 1-hydroxybenzotriazole (HOBT) and 1-dicyclohexylcarbodiimide (DCC) were charged at a temperature of 15-20°C and the reaction mixture was stirred for 8 to 10 hours which forms dicyclohexyl urea (DCU) as a by-product. The by-product dicyclohexyl urea (DCU) was filtered. The obtained filtrate was then extracted twice with aqueous sodium bicarbonate solution. The two layer formed were separated. The organic layer was washed with water and distilled under vacuum at a temperature of 30-60°C to yield the compound of formula II.

The inventors of the present invention have observed that when toluene was used as a reaction solvent in the process of synthesis of the compound of formula II, formation of N-acetyl impurity was completely avoided as ethyl acetate responsible for formation of N-acetyl impurity was not present in the reaction mixture. The comparative study involving use of ethyl acetate and toluene as the reaction solvent in the process for the preparation of the compound of formula II is described in the experimental section.

The reaction of the compound of formula III with the compound of formula IV using 1-hydroxybenzotriazole (HOBT), dicyclohexylcarbodiimide (DCC) and triethylamine, yields the compound of formula II. During the synthesis of the compound of the formula II there the reaction of the compound of formula IV with dicyclohexylcarbodiimide (DCC) takes place which results in the formation of a by-product namely dicyclohexylurea (DCU). This by-product formed in the reaction mixture required to be filtered off. It is important to note here that the by-product, dicyclohexylurea (DCU) is partially soluble in ethyl acetate, the solvent used in the prior art processes, whereas it is insoluble in toluene. When toluene was used as a reaction solvent, the by-product formed was completely filtered off as it is not soluble in toluene thereby providing the filtrate free of the by-product which was further concentrated to give the compound of formula II.

Use of toluene as a solvent in the process for the preparation of the compound of formula II provides additional advantage as it can be recovered easily. As a result the process is rendered cost-effective and industrially viable.

The following examples which fully illustrate the practice of the preferred embodiments of the present invention are intended to be for illustrative purpose only and should not be considered in any way limiting the scope of the present invention.

### Examples

### Example 1:

To a reaction flask the compound of formula III (100g), toluene (800ml) and triethylamine (46.9g) were charged and the reaction mixture was stirred at a temperature of 20-30°C for 30 to 60 minutes. The resulting reaction mixture was then cooled to a temperature of 15-20°C. To the reaction mixture then 1-hydroxybenzotriazole (HOBT) (40.8g), the compound of formula IV (65.2g) and 1-dicyclohexylcarbodiimide (DCC) (62.4g) were charged and the reaction mixture was stirred for 8 to 10 hours at a temperature of 15-20°C, wherein dicyclohexylurea (DCU) is formed as a by-product. The reaction mixture was then filtered and the by-product was washed with toluene. The filtrate was then extracted twice with aqueous sodium bicarbonate solution and the organic layer was separated. The separated organic layer was then washed with water and distilled under vacuum at a temperature of 30-60°C to yield the compound of formula II. Yield 99%, purity 95%.

### Example 2:

To reaction flask the compound of formula III (50g), toluene (500ml) and triethylamine (23.4g) were charged and the reaction mixture was stirred at a temperature of 20-30°C for 30 to 60 minutes. The resulting reaction mixture was then cooled to a temperature of 15-20°C. To the reaction mixture then 1-hydroxybenzotriazole (HOBT) (20.4g), the compound of formula IV (32.6g) and 1-dicyclohexylcarbodiimide (DCC) (31.4g) were charged and the reaction mixture was stirred for 8 to10 hours at a temperature of 15-20°C, wherein dicyclohexylurea (DCU) is formed as a by-product. The reaction mixture was then filtered and the by-product was washed with toluene. The filtrate was then extracted twice with aqueous sodium bicarbonate solution and the organic layer was separated. The separated organic layer was then washed with water and distilled under vacuum at a temperature of 30-60°C to yield the compound of formula II. Yield 99%, purity 95%.

### Comparative study involving process for the preparation of the compound of formula II reported in the prior art wherein ethyl acetate is used as the solvent vis-à-vis the process of the present invention wherein toluene is used as the solvent

**Table-1**

| Ex. No. | Solvent | % of N-acetyl impurity observed in compound of formula II | % of N-acetyl(2S,3aS,7aS)octa hydroindole-2-carboxylic acid observed in the tert-butylamine salt of the compound of formula I | Pharmacopeial limit (EP) |
|---|---|---|---|---|
| 1. | Ethyl acetate | 0.1-0.15 | 0.2-0.3 | 0.1 |
| 2. | Toluene | Not observed | Not observed | 0.1 |

| | | | | |
|---|---|---|---|---|
| Ex. No. = Experiment Number | | | | |

The results presented in above Table-1 are discussed herein below:
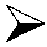 As indicated in Ex. No. 1, when the reaction of the compound of formula III with the compound of formula IV was carried out in presence of ethyl acetate as a solvent, it was observed that N-acetyl impurity is formed with the compound of formula II. About 0.1 to 0.15% N-acetyl impurity is formed in the reaction mixture. Further, when the compound of formula II containing 0.1 to 0.15% of impurity was converted to the compound of formula I (Perindopril) or its tert-butylamine salts, the N-acetyl impurity was converted to an impurity namely N-acetyl(2S,3aS,7aS)octahydroindole-2-carboxylic acid. The percentage of N-acetyl(2S,3aS,7aS)octahydroindole-2-carboxylic acid impurity was found to be 0.2-0.3%. Thus, the presence of N-acetyl(2S,3aS,7aS)octahydroindole-2-carboxylic acid as an impurity with the product ultimately decreases the purity of the therapeutically effective product i.e. the compound of formula I and its tert-butylamine salt.
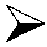 As indicated in Ex. No. 2, when the reaction of the compound of formula III with the compound of formula IV was carried out in the presence of toluene as a solvent, it was observed that the compound of formula II was exclusively formed. In other words this process does not involve formation of the N-acetyl impurity. As a result the purity of the compound of formula I (Perindopril) prepared from the compound of formula II was also increased.

### Details for HPLC analysis:

**Column:** Water Symmetry - C-8, 5 µm, 3.9 x 150 mm Part No - WAT046970
**Mobile Phase:**
   Mobile Phase A:-
      Take 1000 ml water and adjust the pH to 2.5 with Perchloric acid. Filter the mobile phase with 0.45µ nylon membrane filter.
   Mobile Phase B:-
      Take 1000 ml Acetonitrile and add 0.3ml Perchloric acid. Filter the mobile phase with 0.45µ nylon membrane filter.
**Flow:** 1.0 ml/min
**Run Time:** 72 min
**Injection Volume:** 20 µL
**Diluent: -** Mobile phase A: Acetonitrile (9:1)
**Procedure:**
   1) Inject 20µl of the blank i.e. diluent.
   2) Inject 20µl of the Reference solution.
   3) Inject 20µl of the Sample solution.
**Calculation:** Report by area normalization method

## Claims

1. A process for the preparation of a compound of formula II, 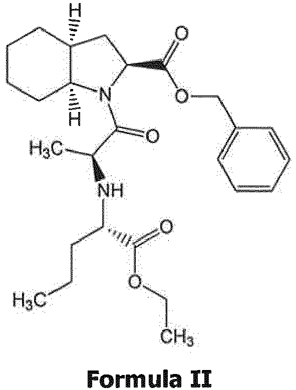 comprising,
reacting the compound of formula III, 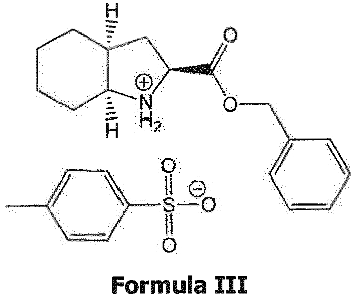 with the compound of formula IV, 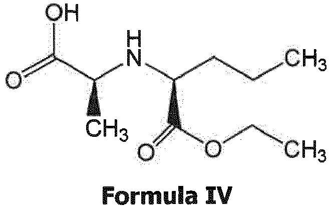 using 1-hydroxybenzotriazole (HOBT), dicyclohexylcarbodiimide (DCC) and triethylamine in the presence of toluene as a solvent at a temperature of 5-40°C to obtain the compound of formula II.

2. The process as claimed in claim 1, wherein toluene is used in an amount ranging from 10 to 15 volumes based on the compound of formula III.

3. The process as claimed in claim 1, wherein the temperature is ranging from 15-20°C.

4. The process as claimed in claim 1, wherein triethylamine is used in an amount ranging from 1 to 3 molar equivalents based on the compound of formula III.

5. The process as claimed in claim 1, wherein hydroxybenzotriazole (HOBT) is used in an amount ranging from 0.8 to 2.5 molar equivalents based on the compound of formula III.

6. The process as claimed in claim 1, wherein dicyclohexylcarbodiimide (DCC) is used in an amount ranging from 0.8 to 2.5 molar equivalents based on the compound of formula III.

## Patentansprüche

1. Verfahren zur Herstellung einer Verbindung der Formel II 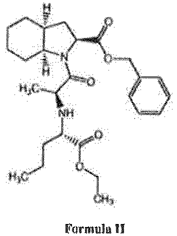 welches umfasst,
Umsetzen der Verbindung der Formel III 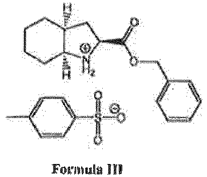 mit der Verbindung der Formel IV, 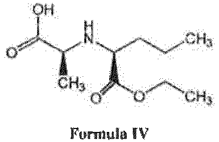 unter Verwendung von 1-Hydroxybenzotriazol (HOBT), Dicyclohexylcarbodiimid (DCC) und Triethylamin in der Anwesenheit von Toluol als Lösungsmittel bei einer Temperatur von 5-40 °C, um die Verbindung der Formel II zu erhalten.

2. Verfahren nach Anspruch 1, wobei Toluol in einer Menge im Bereich von 10 bis 15 Volumina basierend auf der Verbindung der Formel III verwendet wird.

3. Verfahren nach Anspruch 1, wobei die Temperatur im Bereich von 15-20°C ist.

4. Verfahren nach Anspruch 1, wobei Triethylamin in einer Menge im Bereich von 1 bis 3 Molequivalenten basierend auf der Verbindung der Formel III verwendet wird.

5. Verfahren nach Anspruch 1, wobei Hydroxybenzotriazol (HOBT) in einer Menge im Bereich von 0,8 bis 2,5 Molequivalenten basierend auf der Verbindung der Formel III verwendet wird.

6. Verfahren nach Anspruch 1, wobei Dicyclohexylcarbodiimid (DCC) in einer Menge im Bereich von 0,8 bis 2,5 Molequivalenten basierend auf der Verbindung der Formel III verwendet wird.

## Revendications

1. - Procédé pour la préparation d'un composé de formule II : 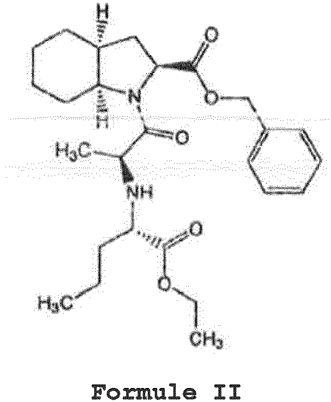 comprenant :
faire réagir le composé de formule III : 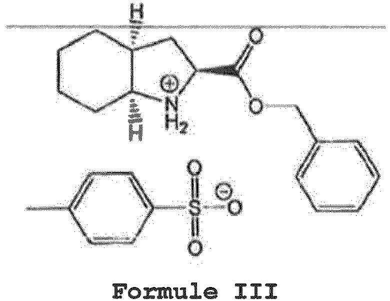 avec le composé de formule IV : 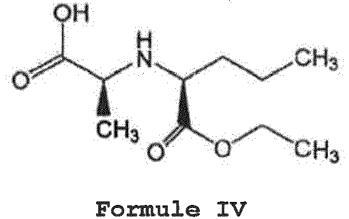 à l'aide du 1-hydroxybenzotriazole (HOBT), du dicyclohexylcarbodiimide (DCC) et de la triéthylamine en présence de toluène comme solvant à une température de 5-40°C pour obtenir le composé de formule II.

2. - Procédé selon la revendication 1, dans lequel le toluène est utilisé dans une quantité se situant dans la plage de 10 à 15 volumes sur la base du composé de formule III.

3. - Procédé selon la revendication 1, dans lequel la température se situe dans la plage de 15-20°C.

4. - Procédé selon la revendication 1, dans lequel la triéthylamine est utilisée dans une quantité se situant dans la plage de 1 à 3 équivalents molaires sur la base du composé de formule III.

5. - Procédé selon la revendication 1, dans lequel de l'hydroxybenzotriazole (HOBT) est utilisé dans une quantité se situant dans la plage de 0,8 à 2,5 équivalents molaires sur la base du composé de formule III.

6. - Procédé selon la revendication 1, dans lequel du dicyclohexylcarbodiimide (DCC) est utilisé dans une quantité se situant dans la plage de 0,8 à 2,5 équivalents molaires sur la base du composé de formule III.
